# EUROPEAN PATENT APPLICATION

(11) **EP 3 345 590 A1**
(43) Date of publication of application: **11.07.2018**
(21) Application number: 16841213.8
(22) Date of filing: 02.06.2016
(51) Int. Cl.: A61K 6/10

(54) **CURABLE PUTTY AND METHOD FOR PRODUCING SAME**

(30) Priority: 04.09.2015 JP 2015175053
(71) Applicant: GC Corporation, Bunkyo-ku Tokyo 113-0033 (JP)
(72) Inventor: KAMINOSONO, Yoshiya, Tokyo 174-8585 (JP); FUSEJIMA, Futoshi, Tokyo 174-8585 (JP)
(74) Representative: Müller-Boré & Partner Patentanwälte PartG mbB
(86) International application number: PCT/JP2016/066440
(87) International publication number: WO 2017/038181

(57) **Abstract**

According to one aspect of the present invention, a hardenable putty used for impression taking includes an inorganic filler at 60% by mass or greater and 85% by mass or less, wherein consistency of the hardenable putty is greater than or equal to 35, and wherein a median diameter of the inorganic filler is greater than or equal to 5 µm and less than or equal to 50 µm on a weight basis.

## Description

### TECHNICAL FIELD

The present invention relates to a hardenable putty used for impression taking and a production method of the hardenable putty.

### BACKGROUND ART

Conventionally, a hardenable putty is used as a dental silicone impression material because of its excellent shaping property. Curable putty generally has a viscosity that can be shaped and shaped while kneading by hand. The hardenable putty generally has a viscosity so as to be able to be mixed by hands and shaped.

Patent Document 1 describes a putty-like hardenable organopolysiloxane composition comprising: (1) 100 parts by weight of an organopolysiloxane including at least two alkenyl groups in one molecule; (2) an amount of supplying a SiH group such that an organohydrogenpolysiloxane including at least three SiH groups in which a hydrogen atom is directly bonded to a silicon atom in one molecule is 0.5 to 5-fold by mole of the alkenyl groups in the ingredient (1); (3) a catalytic amount of a platinum compound; (4) 20 to 600 parts by weight of an inorganic filler; and (5) 5 to 60 parts by weight of an organopolysiloxane including at least 5 mol% of an alkyl group having 7 to 30 carbon atoms within the total organic groups bonded to silicon atoms.

### [Prior Art Document]

### [Patent Document]

[Patent Document 1] Japanese Unexamined Patent Application Publication No. H2-107667

### SUMMARY OF THE INVENTION

### PROBLEMS TO BE SOLVED BY THE INVENTION

However, because the fluidity of the conventional hardenable putty is low, there is a problem that a precise impression cannot be taken when not being used in combination with an impression material having a high fluidity.

In view of the problem of the above described conventional technique, one aspect of the present invention has an object to provide a hardenable putty that has an excellent shaping property and alone enables to take a precise impression.

### MEANS FOR SOLVING THE PROBLEMS

According to one aspect of the present invention, a hardenable putty used for impression taking includes an inorganic filler at 60% by mass or greater and 85% by mass or less, wherein consistency of the hardenable putty is greater than or equal to 35, and wherein a median diameter of the inorganic filler is greater than or equal to 5 µm and less than or equal to 50 µm on a weight basis.

### EFFECTS OF THE INVENTION

According to one aspect of the present invention, it is possible to provide a hardenable putty that has an excellent shaping property and alone enables to take a precise impression.

### EMBODIMENT OF THE INVENTION

Next, an embodiment for implementing the present invention will be described.

A hardenable putty is used for impression taking, and includes an inorganic filler.

A content of the inorganic filler in the hardenable putty is in a range of from 60% to 85% by mass and is preferably in a range of from 65% to 80% by mass. When the content of the inorganic filler in the hardenable putty is less than 60% by mass, the shaping property decreases, and when the content of the inorganic filler in the hardenable putty exceeds 85% by mass, it becomes difficult to obtain a hardenable putty.

The consistency of the hardenable putty is greater than or equal to 35. If the consistency of the hardenable putty is less than 35, it becomes difficult to take a precise impression by the hardenable putty alone. Note that the consistency of the hardenable putty is normally less than 41.

The median diameter of the inorganic filler is in a range of from 5 µm to 50 µm on a weight basis, and is preferably in a range of from 5 µm to 35 µm. When the median diameter of the inorganic filler is less than 5 µm on the weight basis, it becomes difficult to take a precise impression by the hardenable putty alone, and when the median diameter of the inorganic filler exceeds 50 µm on the weight basis, the shaping property of the hardenable putty decreases.

It is preferable that the hardenable putty further includes an organopolysiloxane including two or more alkenyl groups; an organohydrogenpolysiloxane including two or more constituent units represented by a general formula HSiR₂O_{1/2} (where R represents a substituted or unsubstituted hydrocarbon group having 1 to 10 carbon atoms in the formula); a platinum-based catalyst; and a release agent.

Examples of the organopolysiloxane include, but are not limited to, compounds and the like represented by the following general formulas and two or more kinds may be used in combination as the organopolysiloxane. 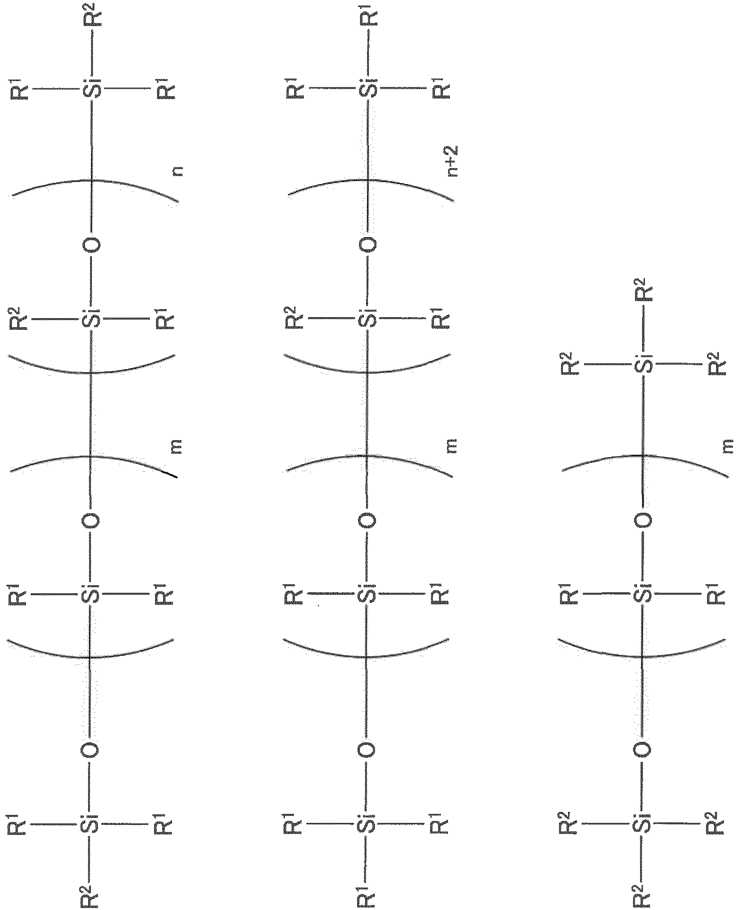 (In the formulas, R¹ is an alkyl group having 1 to 6 carbon atoms such as a methyl group, an ethyl group, a propyl group, or a butyl group, an aryl group, such as a phenyl group or a tolyl group, or a haloalkyl group, such as a chloromethyl group or a 3,3,3-trifluoropropyl group, and R² is an alkenyl group, such as a vinyl group, an allyl group, or a butenyl group.)

It is preferable that the organopolysiloxane includes the following constituent a and the constituent b.

The constituent a includes two or more alkenyl groups and has a viscosity of 1 × 10⁶ mPa·s or more at 25°C. At 25°C, the constituent a is in a putty state and has a polymerization degree of 2000 or more. It is preferable that a vinylsily group is present at an end in the constituent a. In this case, the constituent a may include a plurality of vinyl groups at ends, and may include a vinyl group at a portion other than ends. Further, the constituent a normally includes an alkyl group, such as a methyl group or an ethyl group, or an aryl group, such as a phenyl group or a tolyl group, and preferably includes a methyl group or a phenyl group.

The constituent b includes one or more alkenyl groups and has a viscosity of 100 to 5000 mPa·s or more at 25°C. The constituent b is in a liquid state at 25°C. It is preferable that a vinylsily group is present at an end in the constituent b. In this case, the constituent b may include a plurality of vinyl groups at ends, and may include a vinyl group at a portion other than ends. Further, the constituent b normally includes an alkyl group, such as a methyl group or an ethyl group, or an aryl group, such as a phenyl group or a tolyl group, and preferably includes a methyl group or a phenyl group.

A mass ratio of the constituent b to the constituent a is normally in a range of from 1 to 5 and is preferably in a range of from 1 to 3.

Because the organohydrogenpolysiloxane undergoes a hydrosilylation reaction with the organopolysiloxane, the organohydrogenpolysiloxane acts as a crosslinking agent. In the organohydrogenpolysiloxane, a hydrosilyl group may be present at a portion other than ends, but it is preferable that a hydrosilyl group is present at both ends.

The organohydrogenpolysiloxane preferably includes an alkyl group, an aryl group, such as a phenyl group or a tolyl group, or a haloalkyl group, such as a chloromethyl group or a 3,3,3-trifluoropropyl group, and especially preferably includes a methyl group or a phenyl group.

Two or more kinds of organohydrogenpolysiloxanes may be used in combination.

The organohydrogenpolysiloxane may be either linear, branched, or cyclic.

It is preferable that a molecular weight of the organohydrogenpolysiloxane is lower than a molecular weight of the organopolysiloxane. Thereby, it is possible to enhance the compatibility with the organopolysiloxane.

A mass ratio of the organohydrogenpolysiloxane to the organopolysiloxane is normally in a range of from 0.1% to 30%, and preferably is in a range of from 3% to 20%. When the mass ratio of the organohydrogenpolysiloxane to the organopolysiloxane is greater than or equal to 0.1%, it is possible to enhance the hardness of a hardened product of the hardenable putty and the hardening speed of the hardenable putty, and when the mass ratio of the organohydrogenpolysiloxane to the organopolysiloxane is less than or equal to 30%, it is possible to suppress embrittlement of a hardened product of the hardenable putty.

The platinum-based catalyst is not particularly limited as long as acting as a catalyst for a hydrosilylation reaction. Examples of the platinum-based catalyst include a catalyst in which a carrier, such as silica or carbon black, carries platinum black or platinum, chloroplatinic acid, an alcohol-modified chloroplatinic acid, and a complex of chloroplatinic acid with an olefin. In particular, a vinylsiloxane complex of chloroplatinic acid is preferable.

A mass ratio of the platinum-based catalyst to the total amount of the organopolysiloxane and the organohydrogenpolysiloxane is normally in a range of from 10 ppm to 500 ppm. When the mass ratio of the platinum-based catalyst to the total amount of the organopolysiloxane and the organohydrogenpolysiloxane is greater than or equal to 10 ppm, it is possible to enhance the hardening speed, and when the mass ratio of the platinum-based catalyst to the total amount of the organopolysiloxane and the organohydrogenpolysiloxane is less than or equal to 500 ppm, it is possible to suppress an increase of the production cost.

The platinum-based catalyst is preferably used by being dissolved or dispersed in an alcohol-based, ketone-based, ether-based, or hydrocarbon-based solvent, a polysiloxane oil, or the like.

Note that the hardenable putty may further include various organic nitrogen compounds, organic phosphorus compounds, and acetylene compounds in order to adjust the activity of the platinum-based catalyst.

Because the release agent bleeds on the surface when kneading the hardenable putty, the hardenable putty hardly sticks to hands.

Examples of the release agent may include, but are not limited to, liquid paraffin, white petrolatum, polybutene, and the like.

A mass ratio of the release agent to the organopolysiloxane is normally in a range of from 5% to 60%, and is preferably in a range of from 10% to 40%. When the mass ratio of the release agent to the organopolysiloxane is greater than or equal to 5%, it is possible to enhance the releasing property of the hardenable putty, and when the mass ratio of the release agent to the organopolysiloxane is less than or equal to 60%, it is possible to cause an appropriate amount of the release agent to bleed on the surface of the hardenable putty.

The inorganic filler can enhance an operability of the hardenable putty and a physical property of a hardened product of the hardenable putty.

Examples of the inorganic filler include a glass fiber, fumed silica, a silica powder, such as precipitable silica, powders of quartz, cristobalite, diatomaceous earth, fused quartz, titanium dioxide, carbon, iron oxide, zinc oxide, calcium carbonate, and magnesium carbonate, and the like.

A mass ratio of the inorganic filler to the organopolysiloxane is normally in a range of from 1.8 to 5.0, and is preferably in a range of from 3.0 to 4.0. When the mass ratio of the inorganic filler to the organopolysiloxane is greater than or equal to 1.8, it is possible to enhance the shaping property of the hardenable putty, and when the mass ratio of the inorganic filler to the organopolysiloxane is less than or equal to 5.0, it is possible to enhance the operability of the hardenable putty.

The hardenable putty may further include a pigment, a dye, a perfume, a solvent, a nonionic surfactant, a polyether compound, a silicone resin, and the like.

The hardenable putty can be produced by mixing the inorganic filler, the organopolysiloxane, the organohydrogenpolysiloxane, the platinum-based catalyst, and an aliphatic hydrocarbon.

It is preferable that the hardenable putty is produced by mixing a first paste that includes the organopolysiloxane and the platinum-based catalyst and that does not include the organohydrogenpolysiloxane with a second paste that includes the organopolysiloxane and the organohydrogenpolysiloxane and that does not include the platinum-based catalyst.

Note that a solvent and the inorganic filler may be mixed in the first paste or the second paste, or may be mixed in the first paste and the second paste.

### Examples

In the following, specific examples and comparative examples will be described. The present invention is not limited to these examples. Note that parts mean parts by mass.

### [Example 1]

A first paste was obtained by mixing 100 parts of a mixture in which a mass ratio of dimethyl polysiloxane raw rubber having a viscosity of 1 × 10⁶ mPa·s at 25°C to a dimethylpolysiloxane whose molecular chain both ends are capped by methylvinylsiloxy groups and having a viscosity of 1 × 10³ mPa·s at 25°C is 2 to 3; 10 parts of a methyl hydrogen polysiloxane; 40 parts of a liquid paraffin; and 300 parts of a silica powder whose median diameter is 7 µm on a weight basis.

A second paste was obtained by mixing 100 parts of a mixture in which a mass ratio of dimethyl polysiloxane raw rubber having a viscosity of 1 × 10⁶ mPa·s at 25°C to a dimethylpolysiloxane whose molecular chain both ends are capped by methylvinylsiloxy groups and having a viscosity of 1 × 10³ mPa·s at 25°C is 2 to 3; 2 parts of a silicone oil solution containing 1,3-divinyltetramethyldisiloxane-platinum complex; 40 parts of a liquid paraffin; and 300 parts of a silica powder whose median diameter is 7 µm on a weight basis.

The first paste and the second pate were kneaded 30 seconds at a mass ratio of 1 to 1 to obtain a hardenable putty. The consistency of the hardenable putty was 35.

### [Example 2]

A hardenable putty was obtained in a manner similar to that of the example 1 except that a silica powder whose median diameter is 10 µm on a weight basis was used instead of the silica powder whose median diameter is 7 µm on a weight basis. The consistency of the hardenable putty was 36.

### [Example 3]

A hardenable putty was obtained in a manner similar to that of the example 1 except that a silica powder whose median diameter is 20 µm on a weight basis was used instead of the silica powder whose median diameter is 7 µm on a weight basis. The consistency of the hardenable putty was 38.

### [Comparative Example 1]

A hardenable putty was obtained in a manner similar to that of the example 1 except that a silica powder whose median diameter is 4 µm on a weight basis was used instead of the silica powder whose median diameter is 7 µm on a weight basis. The consistency of the hardenable putty was 32.

### [Comparative Example 2]

A hardenable putty was obtained in a manner similar to that of the example 1 except that a silica powder whose median diameter is 60 µm on a weight basis was used instead of the silica powder whose median diameter is 7 µm on a weight basis.
The consistency of the hardenable putty was 41.

### [Comparative Example 3]

A first paste was obtained by mixing 100 parts of a mixture in which a mass ratio of dimethyl polysiloxane raw rubber having a viscosity of 1 × 10⁶ mPa·s at 25°C to a dimethylpolysiloxane whose molecular chain both ends are capped by methylvinylsiloxy groups and having a viscosity of 1 × 10³ mPa·s at 25°C is 2 to 3; 10 parts of a methyl hydrogen polysiloxane; 40 parts of a liquid paraffin; and 180 parts of a silica powder whose median diameter is 7 µm on a weight basis.

A second paste was obtained by mixing 100 parts of a mixture in which a mass ratio of dimethyl polysiloxane raw rubber having a viscosity of 1 × 10⁶ mPa·s at 25°C to a dimethylpolysiloxane whose molecular chain both ends are capped by methylvinylsiloxy groups and having a viscosity of 1 × 10³ mPa·s at 25°C is 2 to 3; 2 parts of a silicone oil solution containing 1,3-divinyltetramethyldisiloxane-platinum complex; 40 parts of a liquid paraffin; and 180 parts of a silica powder whose median diameter is 7 µm on a weight basis.

The first paste and the second pate were kneaded 30 seconds at a mass ratio of 1 to 1 to obtain a hardenable putty. The consistency of the hardenable putty was 41.

### [Comparative Example 4]

100 parts of a mixture in which a mass ratio of dimethyl polysiloxane raw rubber having a viscosity of 1 × 10⁶ mPa·s at 25°C to a dimethylpolysiloxane whose molecular chain both ends are capped by methylvinylsiloxy groups and having a viscosity of 1 × 10³ mPa·s at 25°C is 2 to 3; 10 parts of a methyl hydrogen polysiloxane; 40 parts of a liquid paraffin; and 950 parts of a silica powder whose median diameter is 7 µm on a weight basis were mixed. However, a pasty mixture could not be obtained and a first paste could not be obtained.

100 parts of a mixture in which a mass ratio of dimethyl polysiloxane raw rubber having a viscosity of 1 × 10⁶ mPa·s at 25°C to a dimethylpolysiloxane whose molecular chain both ends are capped by methylvinylsiloxy groups and having a viscosity of 1 × 10³ mPa·s at 25°C is 2 to 3; 2 parts of a silicone oil solution containing 1,3-divinyltetramethyldisiloxane-platinum complex; 40 parts of a liquid paraffin; and 950 parts of a silica powder whose median diameter is 7 µm on a weight basis were mixed. However, a pasty mixture could not be obtained and a second paste could not be obtained.

### [Consistency]

### [Consistency]

The consistency of each hardenable putty was measured in accordance with JIS T 6513:2005.

The table 1 illustrates characteristics of each hardenable putty.

**Table 1**

| | INORGANIC FILLER | | CONSISTENCY |
|---|---|---|---|
| | MEDIAN DIAMETER ON WEIGHT BASIS [*µ*m] | CONTENT [% BY MASS] | |
| EXAMPLE 1 | 7 | 67 | 35 |
| EXAMPLE 2 | 10 | 67 | 36 |
| EXAMPLE 3 | 20 | 67 | 38 |
| COMPARATIVE EXAMPLE 1 | 4 | 67 | 32 |
| COMPARATIVE EXAMPLE 2 | 60 | 67 | 41 |
| COMPARATIVE EXAMPLE 3 | 7 | 55 | 41 |
| COMPARATIVE EXAMPLE 4 | 7 | 87 | - |

Next, the shaping property, the adhesiveness to hands, and the precision of an impression were evaluated for each hardenable putty.

### [Shaping Property]

Each hardenable putty was placed on an impression material tray of upper jaw L size to evaluate the shaping property. Note that the shaping property was determined as good when the hardenable putty was able to be held in the tray, and the shaping property was determined as poor when the hardenable putty flowed outside the tray.

### [Adhesiveness to Hands]

When the first paste and the second paste were kneaded and when the shaping property of each hardenable putty was evaluated, whether it adhered to hands was checked. Note that the adhesiveness was determined as good when it did not adhere to hands, and the adhesiveness was determined as poor when it adhered to hands.

### [Precision of Impression]

After each hardenable putty was placed on the impression material tray of upper jaw L size, an impression of a resin upper jaw model was taken. After the hardenable putty was hardened, a hardened product of the hardenable putty was taken from the resin upper jaw model to evaluate the precision of the impression. Note that the precision was determined as good when details such as interdental portions of the impression were taken, and the precision was determined as poor when there was an unclear portion in the impression.

The table 2 illustrates evaluation results of the shaping property, the adhesiveness to hands, and the precision of an impression of each hardenable putty.

**Table 2**

| | SHAPING PROPERTY | ADHESIVENESS TO HANDS | PRECISION OF IMPRESSION |
|---|---|---|---|
| EXAMPLE 1 | GOOD | GOOD | GOOD |
| EXAMPLE 2 | GOOD | GOOD | GOOD |
| EXAMPLE 3 | GOOD | GOOD | GOOD |
| COMPARATIVE EXAMPLE 1 | GOOD | GOOD | POOR |
| COMPARATIVE EXAMPLE 2 | POOR | GOOD | GOOD |
| COMPARATIVE EXAMPLE 3 | POOR | GOOD | GOOD |
| COMPARATIVE EXAMPLE 4 | - | - | - |

As can be seen from the table 2, the hardenable putties of the examples 1 to 3 were excellent in the shaping property, the adhesiveness to hands, and the precision of an impression.

In contrast, in the hardenable putty of the comparative example 1, because the median diameter of the inorganic filler was 4 µm on the weight basis and the consistency was 32, the precision of an impression was low.

In the hardenable putty of the comparative example 2, because the median diameter of the silica powder was 60 µm on the weight basis, the shaping property was low.

In the hardenable putty of the comparative example 3, because the content of the inorganic filler was 55% by mass, the shaping property was low.

In the comparative example 4, because the inorganic filler was mixed at the content of 87% by mass, a first paste and a second paste could not be produced, and a hardenable putty could not be obtained.

The present international application is based upon and claims the benefit of priority of Japanese Patent Application No. 2015-175053, filed on September 4, 2015, the entire contents of 2015-175053 are hereby incorporated herein by reference.

## Claims

1. A hardenable putty used for impression taking, the hardenable putty comprising:
an inorganic filler at 60% by mass or greater and 85% by mass or less,
wherein consistency of the hardenable putty is greater than or equal to 35, and
wherein a median diameter of the inorganic filler is greater than or equal to 5 µm and less than or equal to 50 µm on a weight basis.

2. The hardenable putty according to claim 1, further comprising:
an organopolysiloxane including two or more alkenyl groups;
an organohydrogenpolysiloxane including two or more constituent units represented by a general formula HSiR₂O_{1/2} (where R represents a substituted or unsubstituted hydrocarbon group having 1 to 10 carbon atoms in the formula);
a platinum-based catalyst; and
a release agent.

3. A method for producing the hardenable putty according to claim 2, the method comprising:
mixing a first paste that includes the organopolysiloxane and the platinum-based catalyst and that does not include the organohydrogenpolysiloxane with a second paste that includes the organopolysiloxane and the organohydrogenpolysiloxane and that does not include the platinum-based catalyst.
